# EUROPEAN PATENT APPLICATION

(11) **EP 4 166 528 A1**
(43) Date of publication of application: **19.04.2023**
(21) Application number: 21822282.6
(22) Date of filing: 10.06.2021
(51) Int. Cl.: C07C 1/12, C07C 7/148, C07C 9/04, C01B 32/50, B01J 23/42, B01J 23/46

(54) **CO2 METHANATION REACTION APPARATUS PROVIDED WITH SELECTIVE OXIDATION CATALYST FOR CO, AND METOD FOR REMOVING CO FROM GAS**

(30) Priority: 10.06.2020 JP 2020101206
(71) Applicant: Mitsubishi Heavy Industries, Ltd., Tokyo 100-8332 (JP); Mitsubishi Power, Ltd., Nishi-ku, Yokohama-shi Kanagawa 220-8401 (JP)
(72) Inventor: IKEMOTO, Seiji, Yokohama-shi, Kanagawa 220-8401 (JP); SASAKI, Goki, Yokohama-shi, Kanagawa 220-8401 (JP); YOKOYAMA, Koichi, Yokohama-shi, Kanagawa 220-8401 (JP); TANAKA, Yukio, Tokyo 100-8332 (JP); KIYOSAWA, Masashi, Yokohama-shi, Kanagawa 220-8401 (JP)
(74) Representative: Studio Torta S.p.A.
(86) International application number: PCT/JP2021/022186
(87) International publication number: WO 2021/251471

(57) **Abstract**

An object of the present invention is to provide a method and an apparatus for producing a methane-rich gas having a low carbon monoxide concentration, and a method for reducing carbon monoxide from a gas containing carbon monoxide and methane. The method for producing methane-rich gas, comprising adding hydrogen to a gas containing carbon dioxide for carrying out a CO₂ methanation reaction to obtain a gas containing principally methane and collaterally carbon monoxide, and adding oxygen to the above obtained gas to carry out an oxidation reaction of the carbon monoxide in the presence of a CO selective oxidation catalyst, and the method for reducing carbon monoxide from gas containing principally methane and collaterally carbon monoxide, comprising adding oxygen to a gas containing principally methane and collaterally carbon monoxide to carry out an oxidation reaction of the carbon monoxide in the presence of a CO selective oxidation catalyst.

## Description

### TECHNICAL FIELD

The present invention relates to a method and an apparatus for producing a methane-rich gas having a low carbon monoxide concentration and a method for reducing carbon monoxide from a gas containing carbon monoxide and methane.

### BACKGROUND ART

Various techniques for synthesizing methane by reacting carbon dioxide and hydrogen have been proposed for the purpose of fixing carbon dioxide or the like.

For example, Patent Document 1 discloses a production method of methane by hydrogenation of CO₂, comprising adding H₂ to a gas containing CO₂ in the presence of a catalyst with a reaction start temperature of 200 to 550 °C and a reaction pressure of atmospheric pressure, wherein the catalyst comprises a porous support made of an inorganic oxide and rhodium supported on the porous support and the (111) plane of the rhodium is exposed.

Patent document 2 discloses a methanation reaction apparatus for producing a gas containing methane by supplying hydrogen to a source gas containing carbon dioxide, characterized in that the apparatus comprises: a first reactor that supplies the source gas and a part of hydrogen; a second reactor that supplies balance of the hydrogen to the gas mixture to be reacted coming from the first reactor and adjusts an amount of the hydrogen required for a reaction; and a third reactor that adjusts a composition ratio of the generated gas coming from the second reactor; wherein a reaction temperature of the first reactor is modulated by adjusting the amount of the hydrogen supplied to the first reactor.

Patent Document 3 discloses a method for synthesizing methane from carbon dioxide and hydrogen, characterized in that the method comprises a first reaction step for reacting carbon dioxide and hydrogen to obtain carbon monoxide; and a second reaction step for reacting the carbon monoxide generated in the first reaction step and hydrogen to obtain methane.

Patent Document 4 discloses a method for producing methane from a gas containing carbon dioxide and hydrogen using a catalyst, characterized in that the method comprises a first methanation reaction (CO₂ + 4H₂ --> CH₄ + 2H₂O) step; a subsequent shift reaction (CO + H₂O --> CO₂ + H₂) step and a subsequent second methanation reaction step. The shift reaction is carried out at a temperature around 220 °C while water is added.

### CITATION LIST

### PATENT LITERATES

Patent Document 1 : JP H06-142513 A
Patent Document 2 : JP 2013-136538 A
Patent Document 3 : JP 2012-140382 A
Patent Document 4 : JP 2015-124217 A

### SUMMARY OF THE INVENTION

### PROBLEMS TO BE SOLVED BY THE INVENTION

Synthetic methane obtained by reacting carbon dioxide and hydrogen contains not a little H₂, CO₂, CO and the like. In particular, CO is known as a toxic gas. When synthetic methane is used, for example, as a raw material for city gas, it is necessary to reduce a cost of separating and recovering unreacted residual components (H₂, CO₂, CO) .

An object of the present invention is to provide a method and an apparatus for producing a methane-rich gas having a low carbon monoxide concentration, and a method for reducing carbon monoxide from a gas containing carbon monoxide and methane.

### MEANS TO SOLVE THE PROBLEMS

Studies to solve the above problems have resulted in the present invention including the following aspects.
[1] A method for producing methane-rich gas, comprising :
   adding hydrogen to a gas containing carbon dioxide for carrying out a CO₂ methanation reaction to obtain a gas containing principally methane and collaterally carbon monoxide, and
   adding oxygen to the obtained gas to carry out an oxidation reaction of the carbon monoxide in the presence of a CO selective oxidation catalyst.
[2] A method for reducing carbon monoxide from gas containing principally methane and collaterally carbon monoxide, comprising
   adding oxygen to a gas containing principally methane and collaterally carbon monoxide to carry out an oxidation reaction of the carbon monoxide in the presence of a CO selective oxidation catalyst.
[3] An apparatus for producing methane-rich gas, comprising
   a CO₂ methanation reaction device,
   a line configured to feed a gas containing carbon dioxide into the CO₂ methanation reaction device,
   a line configured to feed hydrogen into the CO₂ methanation reaction device,
   a CO selective oxidation reaction device,
   a line configured to feed oxygen into the CO selective oxidation reaction device, and
   a line configured to feed a gas generated in the CO₂ methanation reaction device into the CO selective oxidation reaction device.
[4] The apparatus for producing methane-rich gas according to [3], further comprising,
   a device for cooling the gas generated in the CO₂ methanation reaction device in the middle of the line configured to feed the gas generated in the CO₂ methanation reaction device into the CO selective oxidation reaction device.

### ADVANTAGEOUS EFFECTS OF THE INVENTION

According to the method of the present invention, carbon monoxide can be reduced from a gas containing carbon monoxide and methane, and a methane-rich gas with low carbon monoxide concentration can be produced. The apparatus of the present invention is suitable for producing methane-rich gas.

### BRIEF DESCRIPTION OF THE DRAWINGS

Fig. 1 is a diagram showing an example of the CO selective oxidation reaction device.
Fig. 2 is a diagram showing an example of the methane-rich gas production apparatus.
Fig. 3 is a diagram showing another example of the methane-rich gas production apparatus.
Fig. 4 is a diagram showing an example of a device for using an SOFC exhaust gas as the CO₂ source.

### EMBODIMENTS FOR CARRYING OUT THE INVENTION

Embodiments of the present invention are shown below to describe the present invention more specifically. These are merely examples for explanation, and the present invention is not limited by these embodiments.

The method for producing methane-rich gas of the present invention, comprises adding hydrogen to a gas containing carbon dioxide for carrying out a CO₂ methanation reaction to obtain a gas containing principally methane and collaterally carbon monoxide, and adding oxygen to the obtained gas to carry out an oxidation reaction of the carbon monoxide in the presence of a CO selective oxidation catalyst.

An apparatus for producing methane-rich gas of the present invention, comprises a CO₂ methanation reaction device and a CO selective oxidation reaction device, and further comprises a line configured to feed a gas containing carbon dioxide into the CO₂ methanation reaction device, a line configured to feed hydrogen into the CO₂ methanation reaction device, a line configured to feed oxygen into the CO selective oxidation reaction device, and a line configured to feed a gas generated in the CO₂ methanation reaction device into the CO selective oxidation reaction device.

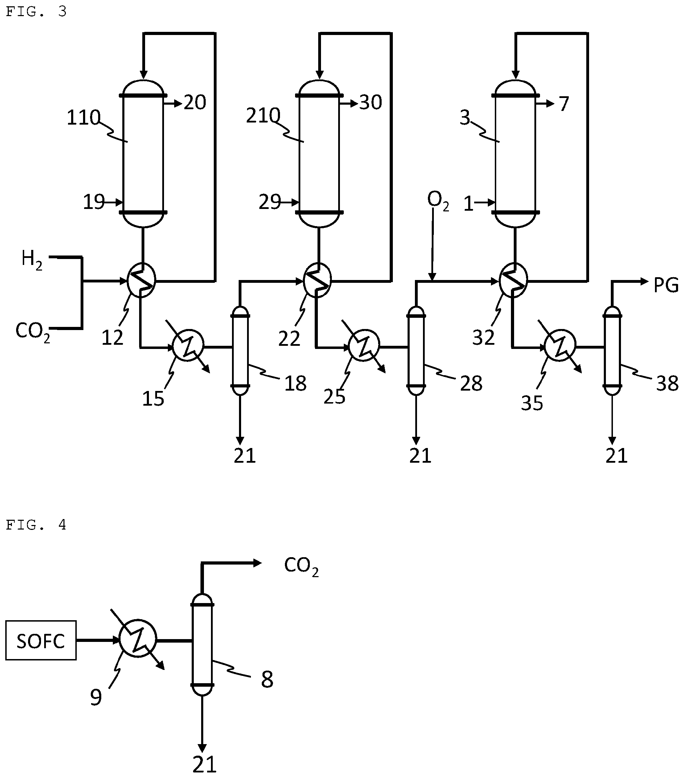

The gas containing carbon dioxide can be a gas discharged from a thermal power plant, an ironwork, or the like, a gas discharged from a fuel cell such as Solid Oxide Fuel Cell (SOFC) or the like. As shown in FIG. 4, the gas discharged from the fuel cell can be cooled by the cooler 9 and separated from the drain 21 by the drain separator 8, to be used as a CO₂ source.

A gas containing hydrogen may be a gas obtained by electrolysis of water, a gas obtained by reforming methane gas or methanol generated from biomass or fossil fuels such as oil and natural gas, or a gas secondarily produced in a steel plant, a chemical factory or the like. Hydrogen may be produced using surplus power generated by electric generation using renewable energy.

The CO₂ methanation reaction is a chemical reaction (CO₂ + 4H₂ --> CH₄ + 2H₂O) that produces methane from carbon dioxide and hydrogen. A temperature in the CO₂ methanation reaction is usually 200 to 500 °C, preferably 220 to 400 °C, more preferably 250 to 300 °C. A pressure during the CO₂ methanation reaction is not particularly limited, but is preferably normal pressure (NP: about 0.1 MPa) to 0.9 MPa.

In the CO₂ methanation reaction, a chemical reaction that produces carbon monoxide (CO₂ + H₂ --> CO + H₂O) or a chemical reaction that produces methanol (CO₂ + 3H₂ --> CH₃OH + H₂O) may occur concomitantly.

The CO₂ methanation reaction device comprises a reactor having an inlet port for a gas containing carbon dioxide, an inlet port for a gas containing hydrogen and an outlet port for a reaction product that is a gas containing primarily methane and collaterally carbon monoxide. The reactor can be tubular type reactor or tank type reactor. The reactor usually has facilities for adjusting the temperature in the reactor. The facilities for temperature adjustment usually comprises a jacket or a heat transfer tube for heat exchange with the reaction gas, a control device for controlling the amount of heat exchanged and a measuring device for measuring the temperature in the reactor.

A methanation catalyst can be used for the CO₂ methanation reaction. As the methanation catalyst, mentioned can be Ni-based catalyst, platinum group metal-based catalyst, other noble metal-based catalyst, and the like. Specific examples of the methanation catalyst can include nickel aluminate (NiAlₓO_{y}), Ru/NiAlₓO_{y}, Ru/Al₂O₃, Ru/TiO₂, Ni/TiO₂ and Ru-Ni/TiO₂. The methanation catalyst can be appropriately used in the form of powder, granule, pellet, flat plate, corrugated or pleated plate, corrugated board, honeycomb and the like. The methanation catalyst may be loaded or packed in an inner cavity of the reactor or be adhered to the inside wall of the inner cavity of the reactor.

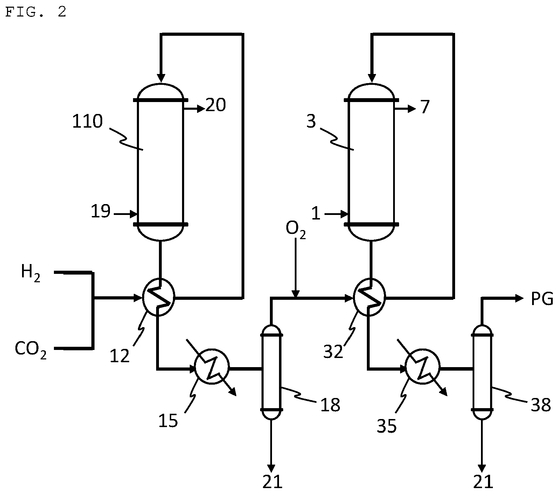

The CO₂ methanation reaction can be carried out in only one CO₂ methanation reactor connected as shown in FIG. 2, or in a plurality of CO₂ methanation reactors connected in series as shown in FIG. 3. Also, the CO₂ methanation reaction may be carried out in a plurality of CO₂ methanation reactors connected in parallel.

The gas produced by the CO₂ methanation reaction contains not a little carbon monoxide. Carbon monoxide concentration in the gas is not particularly limited, but may be not less than 100 ppm. In the present invention, carbon monoxide in the gas produced by the CO₂ methanation reaction is oxidized (2CO + O₂ --> 2CO₂) by adding oxygen in the presence of a CO selective oxidation catalyst. An upper limit of a temperature in the CO selective oxidation reaction is usually 200 °C, preferably 150 °C, more preferably 120 °C, still more preferably 100 °C, and a lower limit of that is usually 20 °C, preferably 35 °C, more preferably 50 °C, still more preferably 80 °C. As the temperature in the CO selective oxidation reaction increases, the oxidation reaction of methane tends to occur more easily. The addition of oxygen is preferably carried out so that a molar ratio of oxygen (O₂) to carbon monoxide (CO) is 2 to 6. The pressure during the CO selective oxidation reaction is not particularly limited, but is preferably normal pressure (NP : about 0.1 MPa) to 0.9 MPa.

Also, the gas produced by the CO₂ methanation reaction may contain hydrogen and water. Some of the hydrogen is chemically changed into water by the addition of oxygen.

The CO selective oxidation reaction device comprises a reactor having an inlet port for the gas produced in the CO₂ methanation reaction, an inlet port for the oxygen-containing gas and an outlet port for the reaction product that is a gas comprising mainly methane. The reactor can be tubular type reactor or tank type reactor. The reactor usually has facilities for controlling the temperature in the reactor. The facilities for temperature control usually comprises a jacket or heat transfer tube for heat exchange with the reaction gas, a control device for adjusting the amount of heat exchanged, and a measuring device for measuring the temperature in the reactor. In addition, for the temperature controlling, a device (such as a heat exchanger) for cooling the gas produced by the CO₂ methanation reaction device is preferably installed in the middle of the line configured to supply the gas produced by the CO₂ methanation reaction device to the CO selective oxidation reaction device. Water (H₂O) can be removed by this cooling, and the temperature can be adjusted to suit the CO selective oxidation reaction.

As the CO selective oxidation catalyst used in the CO selective oxidation reaction, mentioned can be an oxide based catalyst, a platinum group metal (Pt, Ru, Rh, Pd, etc.) based catalyst, another noble metal based catalyst, photocatalyst, or the like. Specific examples of the CO selective oxidation catalyst can include Ru/Al₂O₃, Ru/C, Coₓ-Fe₂O, Co₃O₄, Cu/CeO₂-ZrO₂, Ni/CeO₂-ZrO₂, Co/CeO₂-ZrO₂, Fe/CeO₂-ZrO₂, Pt/Al₂O₃, CuMn₂O₄, CuZnO, Pt/SiO₂, Pd/Al₂O₃, Pt/SnO₂, Pd/CeO₂, Pt/TiO₂, PdCl₂-CuCl₂/C, Au/TiO₂ and Au/Fe₂O₃. The CO selective oxidation catalyst can be used in the shape of powder, granule, pellet, flat plate, pleated or corrugated plate, corrugated board, or honeycomb as appropriate. The CO selective oxidation catalyst may be a supported catalyst in which an element having an oxidizing function such as Pt, Ru, Rh and Pd is supported on a carrier such as Al₂O₃, SiO₂, TiO₂ and ZrO₂. The CO selective oxidation catalyst may be loaded or packed in an inner cavity of the reactor, or may be adhered to the inside wall of the inner cavity of the reactor.

The CO selective oxidation reaction is preferably carried out so that the resulting methane-rich gas has a carbon monoxide concentration of 30 ppm or less. A space velocity (GHSV) in the reactor of the CO selective oxidation reaction device is preferably 4,000 to 150,000 hr⁻¹, more preferably 5,000 to 10,000 hr⁻¹.

The CO-selective oxidation reaction can reduce carbon monoxide from a gas containing principally methane and collaterally carbon monoxide, resulting in obtaining a methane-rich gas.

The methane-rich gas obtained by the present invention can be supplied as fuel to a gas turbine. Electric power can be generated by this gas turbine. Since a flue gas from the gas turbine usually contains carbon dioxide, the flue gas can be used as a CO₂ source in the above method for producing methane-rich gas.

The carbon monoxide removal performance of the method of the present invention is demonstrated by the following experimental examples.

### [Experimental Examples]

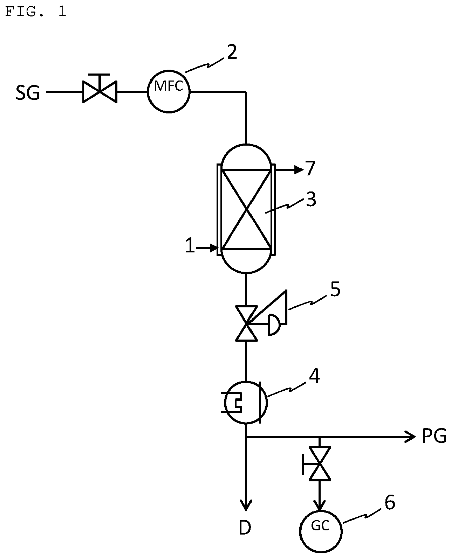

A CO selective oxidation reaction device shown in FIG. 1 was equipped.

Each of a catalyst (Ru/Al₂O₃, Ru content 0.40%) in which ruthenium was supported on γ-alumina, a catalyst (Pt/Al₂O₃, Pt content 0.40%) in which platinum was supported on γ-alumina and a catalyst (Rh/Al₂O₃, Rh content 0.40%) in which rhodium was supported on γ-alumina was sieved to a particle size range of 0.1 mm to 0.2 mm to be charged into a CO selective oxidation reactor.

The source gas SG containing the components shown in Tables 1 to 4 was supplied to the reactor at the inlet temperature, pressure and space velocity shown in Tables 1 to 4. The components of the produced gas PG were analyzed using a gas chromatography (GL-4000 produced by GL-Science). The results are shown in Tables 1 to 4.

### [TAB. 1]

**Table 1**

| | | 1 | 2 | 3 | 4 | 5 | 6 | 7 |
|---|---|---|---|---|---|---|---|---|
| Source gas | | | | | | | | |
| | O₂ [ppm] | 2,000 | 4,000 | 2,000 | 6,000 | 0 | 2,000 | 2,000 |
| | CO [ppm] | 1, 000 | 1, 000 | 1,000 | 1, 000 | 1,000 | 1, 000 | 1,000 |
| | CH₄ [vol%] | 92.2 | 92.0 | 92.2 | 91.8 | 92.4 | 92.2 | 92.2 |
| | H₂ [vol%] | 6.0 | 6.0 | 6.0 | 6.0 | 6.0 | 6.0 | 6.0 |
| | CO₂ [vol%] | 1.5 | 1.5 | 1.5 | 1.5 | 1.5 | 1.5 | 1.5 |
| | Total [vol%] | 100.0 | 100.0 | 100.0 | 100.0 | 100.0 | 100.0 | 100.0 |
| | O₂/CO [mol ratio] | 2 | 4 | 2 | 6 | 0 | 2 | 10 |

| Reactor | | | | | | | | |
|---|---|---|---|---|---|---|---|---|
| | Inlet Temp. [°C] | 100 | 100 | 80 | 80 | 100 | 150 | 100 |
| | Pressure[MPa] | 0.1 (NP) | 0.1 (NP) | 0.1 (NP) | 0.1 (NP) | 0.1 (NP) | 0.1 (NP) | 0.1 (NP) |
| | GHSV[h⁻¹] | 5,000 | 5, 000 | 10,000 | 10,000 | 5, 000 | 5, 000 | 5,000 |
| | Catalyst | Ru /Al₂O₃ | Ru /Al₂O₃ | Ru /Al₂O₃ | Pt /Al₂O₃ | Ru /Al₂O₃ | Pt /Al₂O₃ | Ru /Al₂O₃ |

| Produced gas | | | | | | | | |
|---|---|---|---|---|---|---|---|---|
| | CO [ppm] | 10 | 7 | 5 | 3 | 1, 000 | 352 | 52 |
| | CH₄ [vol%] | 92.6 | 93.0 | 93.0 | 93.7 | 92.4 | 92.6 | 93.8 |
| | H₂ [vol%] | 5.6 | 5.2 | 5.0 | 4.6 | 6.0 | 5.5 | 4.5 |
| | CO₂ [vol%] | 1.6 | 1.6 | 1.6 | 1.6 | 1.5 | 1.6 | 1.6 |
| | Total [vol%] | 99.80 | 99.83 | 99.60 | 99.90 | 100.00 | 99.74 | 99.91 |
| | COChangeRatio[%] | -99.0 | -99.3 | -99.5 | -99.7 | 0.0 | -64.8 | -94.8 |
| | H₂ChangeRatio[%] | -6.8 | -13.3 | -16.5 | -23.2 | 0.0 | -8.3 | -25.0 |
| | CH₄ChangeRatio [%] | 0.4 | 1.1 | 0.9 | 2.1 | 0.0 | 0.4 | 1.7 |

### [TAB. 2]

**Table 2**

| | | 8 | 9 | 10 | 11 | 12 | 13 | 14 |
|---|---|---|---|---|---|---|---|---|
| Source gas | | | | | | | | |
| | O₂ [ppm] | 2,000 | 2,000 | 6,000 | 2,000 | 4,000 | 4,000 | 0 |
| | CO [ppm] | 1, 000 | 1, 000 | 1,000 | 1, 000 | 1,000 | 1, 000 | 1,000 |
| | CH₄ [vol%] | 92.2 | 92.2 | 91.8 | 92.2 | 92.0 | 92.0 | 92.4 |
| | H₂ [vol%] | 6.0 | 6.0 | 6.0 | 6.0 | 6.0 | 6.0 | 6.0 |
| | CO₂ [vol%] | 1.5 | 1.5 | 1.5 | 1.5 | 1.5 | 1.5 | 1.5 |
| | Total [vol%] | 100.0 | 100.0 | 100.0 | 100.0 | 100.0 | 100.0 | 100.0 |
| | O₂/CO [mol ratio] | 2 | 2 | 6 | 2 | 4 | 4 | 0 |

| Reactor | | | | | | | | |
|---|---|---|---|---|---|---|---|---|
| | Inlet Temp. [°C] | 100 | 100 | 100 | 80 | 80 | 150 | 100 |
| | Pressure[MPa] | 0.1 (NP) | 0.4 | 0.4 | 0.4 | 0.4 | 0.4 | 0.4 |
| | GHSV[h⁻¹] | 1, 000 | 5, 000 | 5, 000 | 10,000 | 10,000 | 5, 000 | 5,000 |
| | Catalyst | Ru /Al₂O₃ | Ru /Al₂O₃ | Ru /Al₂O₃ | Rh /Al₂O₃ | Ru /Al₂O₃ | Rh /Al₂O₃ | Ru /Al₂O₃ |

| Produced gas | | | | | | | | |
|---|---|---|---|---|---|---|---|---|
| | CO [ppm] | 212 | 9 | 5 | 2 | 1 | 10 | 1,000 |
| | CH₄ [vol%] | 92.5 | 92.8 | 93.5 | 93.1 | 93.8 | 93.8 | 92.4 |
| | H₂ [vol%] | 5.8 | 5.3 | 4.6 | 5.0 | 4.5 | 4.5 | 6.0 |
| | CO₂ [vol%] | 1.6 | 1.6 | 1.6 | 1.6 | 1.6 | 1.7 | 1.5 |
| | Total [vol%] | 99.92 | 99.80 | 99.73 | 99.68 | 99.90 | 100.00 | 100.00 |
| | COChangeRatio[%] | -78.8 | -99.1 | -99.5 | -99.8 | -99.9 | -99.0 | 0.0 |
| | H₂ChangeRatio[%] | -3.3 | -11.2 | -23.3 | -17.0 | -25.0 | -25.0 | 0.0 |
| | CH₄ChangeRatio [%] | 0.3 | 0.7 | 1.9 | 1.0 | 2.0 | 2.0 | 0.0 |

### [TAB. 3]

**Table 3**

| | | 15 | 16 | 17 | 18 | 19 | 20 | 21 |
|---|---|---|---|---|---|---|---|---|
| Source gas | | | | | | | | |
| | O₂ [ppm] | 1,000 | 2,000 | 2,000 | 2,000 | 2,000 | 2,000 | 4,000 |
| | CO [ppm] | 1, 000 | 1, 000 | 1,000 | 1, 000 | 1,000 | 1, 000 | 1,000 |
| | CH₄ [vol%] | 92.3 | 92.2 | 92.2 | 92.2 | 92.2 | 92.2 | 92.0 |
| | H₂ [vol%] | 6.0 | 6.0 | 6.0 | 6.0 | 6.0 | 6.0 | 6.0 |
| | CO₂ [vol%] | 1.5 | 1.5 | 1.5 | 1.5 | 1.5 | 1.5 | 1.5 |
| | Total [vol%] | 100.0 | 100.0 | 100.0 | 100.0 | 100.0 | 100.0 | 100.0 |
| | O₂/CO [mol ratio] | 1 | 2 | 2 | 10 | 2 | 2 | 4 |

| Reactor | | | | | | | | |
|---|---|---|---|---|---|---|---|---|
| | Inlet Temp. [°C] | 100 | 120 | 150 | 100 | 100 | 100 | 100 |
| | Pressure[MPa] | 0.4 | 0.4 | 0.4 | 0.4 | 0.4 | 0.9 | 0.9 |
| | GHSV[h⁻¹] | 5,000 | 5, 000 | 5, 000 | 5, 000 | 1, 000 | 5, 000 | 5,000 |
| | Catalyst | Ru /Al₂O₃ | Rh /Al₂O₃ | Rh /Al₂O₃ | Ru /Al₂O₃ | Ru /Al₂O₃ | Ru /Al₂O₃ | Ru /Al₂O₃ |

| Produced gas | | | | | | | | |
|---|---|---|---|---|---|---|---|---|
| | CO [ppm] | 127 | 44 | 252 | 41 | 153 | 8 | 4 |
| | CH₄ [vol%] | 92.6 | 93.0 | 93.0 | 93.9 | 92.5 | 93.3 | 93.9 |
| | H₂ [vol%] | 5.7 | 5.2 | 5.2 | 4.4 | 5.7 | 5.0 | 4.3 |
| | CO₂ [vol%] | 1.6 | 1.7 | 1.6 | 1.6 | 1.6 | 1.6 | 1.6 |
| | Total [vol%] | 99.87 | 99.92 | 99.83 | 99.90 | 99.82 | 99.9 | 99.8 |
| | COChangeRatio[%] | -87.3 | -95.6 | -74.8 | -95.9 | -84.7 | -99.2 | -99.6 |
| | H₂ChangeRatio[%] | -5.7 | -13.0 | -13.3 | -26.7 | -5.0 | -16.7 | -28.3 |
| | CH₄ChangeRatio [%] | 0.3 | 0.9 | 0.9 | 1.8 | 0.3 | 1.2 | 2.1 |

### [TAB. 4]

**Table 4**

| | | 22 | 23 | 24 | 25 | 26 | 27 |
|---|---|---|---|---|---|---|---|
| Source gas | | | | | | | |
| | O₂ [ppm] | 2,000 | 6,000 | 0 | 2,000 | 2,000 | 2,000 |
| | CO [ppm] | 1, 000 | 1, 000 | 1, 000 | 1, 000 | 1, 000 | 1,000 |
| | CH₄ [vol%] | 92.2 | 91.8 | 92.4 | 92.2 | 92.2 | 92.2 |
| | H₂ [vol%] | 6.0 | 6.0 | 6.0 | 6.0 | 6.0 | 6.0 |
| | CO₂ [vol%] | 1.5 | 1.5 | 1.5 | 1.5 | 1.5 | 1.5 |
| | Total[vol%] | 100.0 | 100.0 | 100.0 | 100.0 | 100.0 | 100.0 |
| | O₂/CO [mol ratio] | 2 | 6 | 0 | 2 | 10 | 2 |

| Reactor | | | | | | | |
|---|---|---|---|---|---|---|---|
| | Inlet Temp. [°C] | 80 | 80 | 100 | 150 | 100 | 100 |
| | Pressure[MPa] | 0.9 | 0.9 | 0.9 | 0.9 | 0.9 | 0.9 |
| | GHSV[h⁻¹] | 10,000 | 10,000 | 5, 000 | 5, 000 | 5, 000 | 1,000 |
| | Catalyst | Ru /Al₂O₃ | Ru /Al₂O₃ | Ru /Al₂O₃ | Ru /Al₂O₃ | Ru /Al₂O₃ | Ru /Al₂O₃ |

| Produced gas | | | | | | | |
|---|---|---|---|---|---|---|---|
| | CO [ppm] | 2 | 1 | 1, 000 | 203 | 35 | 111 |
| | CH₄ [vol%] | 93.3 | 93.9 | 92.4 | 93.2 | 94.0 | 92.7 |
| | H₂ [vol%] | 4.7 | 4.1 | 6.0 | 5.0 | 3.9 | 5.5 |
| | CO₂ [vol%] | 1.6 | 1.6 | 1.5 | 1.6 | 1.6 | 1.6 |
| | Total[vol%] | 99.6 | 99.6 | 100.0 | 99.8 | 99.5 | 99.8 |
| | COChangeRatio[%] | -99.8 | -99.9 | 0.0 | -79.7 | -96.5 | -88.9 |
| | H₂ChangeRatio[%] | -21.7 | -31.7 | 0.0 | -16.7 | -35.0 | -8.3 |
| | CH₄ChangeRatio [%] | 1.2 | 2.3 | 0.0 | 1.1 | 2.0 | 0.5 |

### CODE EXPLANATION

- 1:: Heat transfer medium inflow path
- 2:: Mass flow meter
- 3:: CO selective oxidation reactor
- 4:: Cooler
- 5:: Back pressure regulator
- 6:: Gas chromatography
- 7:: Heat transfer medium outflow path
- PG:: Methane rich gas
- SG:: Source gas
- D:: Drain

- 19:: First heat transfer medium inflow path
- 20:: First heat transfer medium outflow path
- 29:: Second heat transfer medium inflow path
- 30:: Second heat transfer medium outflow path
- 110:: First CO₂ methanation reactor
- 210:: Second CO₂ methanation reactor
- 12, 22, 32:: Heat exchanger
- 9, 15, 25, 35:: Cooler
- 8, 18, 28, 38:: Drain separator
- 21:: Drain

## Claims

1. A method for producing methane-rich gas, comprising adding hydrogen to a gas containing carbon dioxide for carrying out a CO₂ methanation reaction to obtain a gas containing principally methane and collaterally carbon monoxide, and
adding oxygen to the obtained gas to carry out an oxidation reaction of the carbon monoxide in the presence of a CO selective oxidation catalyst.

2. A method for reducing carbon monoxide from gas containing principally methane and collaterally carbon monoxide, comprising
adding oxygen to a gas containing principally methane and collaterally carbon monoxide to carry out an oxidation reaction of the carbon monoxide in the presence of a CO selective oxidation catalyst.

3. An apparatus for producing methane-rich gas, comprising
a CO₂ methanation reaction device,
a line configured to feed a gas containing carbon dioxide into the CO₂ methanation reaction device,
a line configured to feed hydrogen into the CO₂ methanation reaction device,
a CO selective oxidation reaction device,
a line configured to feed oxygen into the CO selective oxidation reaction device, and
a line configured to feed a gas generated in the CO₂ methanation reaction device into the CO selective oxidation reaction device.

4. The apparatus for producing methane-rich gas according to claim 3, further comprising
a device for cooling the gas generated in the CO₂ methanation reaction device in the middle of the line configured to feed the gas generated in the CO₂ methanation reaction device into the CO selective oxidation reaction device.
